# EUROPEAN PATENT APPLICATION

(11) **EP 3 441 070 A1**
(43) Date of publication of application: **13.02.2019**
(21) Application number: 17779236.3
(22) Date of filing: 07.04.2017
(51) Int. Cl.: A61K 31/506, A61P 3/12

(54) **DRUG FOR PREVENTING OR TREATING LACTIC ACIDOSIS**

(30) Priority: 08.04.2016 JP 2016078492
(71) Applicant: Keio University, Tokyo 108-8345 (JP); Daiichi Sankyo Company, Limited, Tokyo 103-8426 (JP)
(72) Inventor: MINAMISHIMA, Yoji, Tokyo 160-8582 (JP); TORAMARU, Tomoko, Tokyo 160-8582 (JP); SUHARA, Tomohiro, Tokyo 160-8582 (JP); YAMAGUCHI, Kyoji, Tokyo 140-8710 (JP)
(74) Representative: Krauss, Jan
(86) International application number: PCT/JP2017/014507
(87) International publication number: WO 2017/175859

(57) **Abstract**

An object is to provide a pharmaceutical composition containing a compound having an excellent effect of preventing or treating lactic acidosis. Provided is a pharmaceutical composition for preventing or treating lactic acidosis, including, as an active ingredient, a compound represented by the general formula (I) or a pharmacologically acceptable salt thereof: 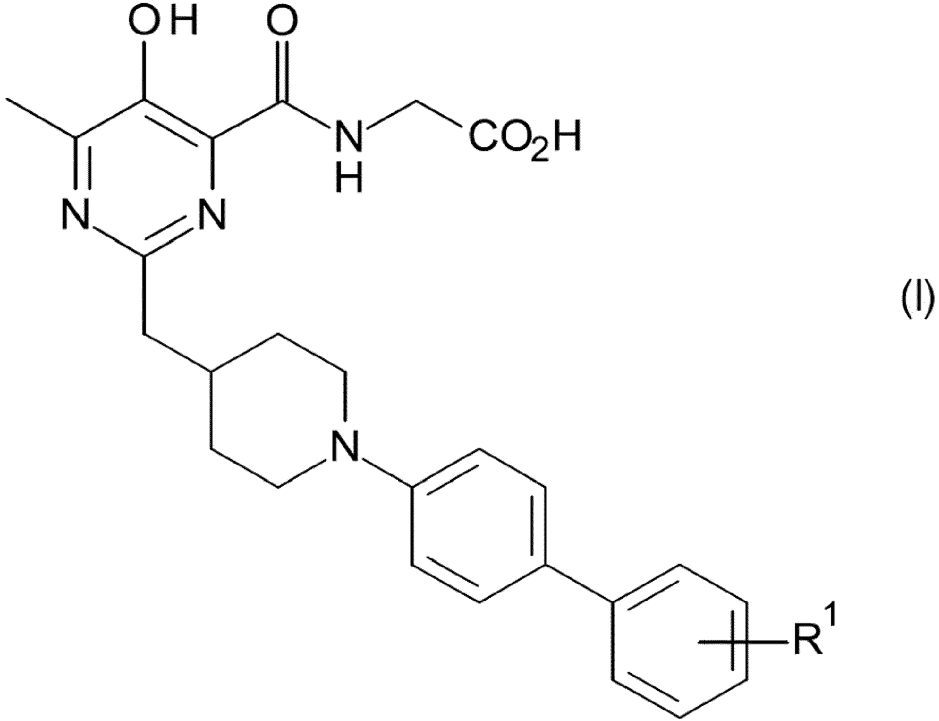 in the general formula (I), R¹ represents a hydroxy C₁ to C₆ alkyl group, a C₂ to C₇ alkanoyl group, a C₂ to C₇ alkanoyl C₁ to C₆ alkyl group, a (C₁ to C₆ alkoxy) carbonyl group, a (C₁ to C₆ alkoxy) carbonyl C₁ to C₆ alkyl group, a carboxy group, or a carboxy C₁ to C₆ alkyl group.

## Description

### Technical Field

The present invention relates to a pharmaceutical composition for preventing or treating lactic acidosis, containing a 5-hydroxypyrimidine-4-carboxamide derivative as an active ingredient.

The present application claims priority from Japanese Patent Application No. 2016-078492, which is incorporated herein by reference.

### Background Art

Lactic acidosis is a serious condition with a high mortality rate, and has been reported in ischemic diseases or severe infections including sepsis, and in diabetic patients who have received a prescription of metformin, which is a biguanide drug that lowers a blood sugar level by suppressing hepatic gluconeogenesis, in particular, chronic kidney disease (hereinafter sometimes referred to as CKD) patients.

In addition to lowering a blood sugar level, metformin suppresses growth of cancer cells, reduces an incidence of cancer, and is also associated with longevity. Therefore, the number of patients taking metformin and an incidence of metformin-associated lactic acidosis (hereinafter sometimes referred to as MALA) are considered to increase in the future.

Metformin is one of the most general methods of treating type 2 diabetes, and has an effect of reducing a blood sugar level by suppressing hepatic gluconeogenesis via a plurality of mechanisms including mitochondrial complex I, glycerol-3-phosphate dehydrogenase (GPDH), and AMP-activated protein kinase (AMPK) (see Non Patent Literature 1).

Metformin has been prescribed to 120,000,000 or more people worldwide (see Non Patent Literature 2), and the number of patients with impaired glucose tolerance is expected to increase to 420, 000, 000 by 2025 (see Non Patent Literature 3). However, it is reported that metformin is associated with serious lactic acidosis, and an overall incidence thereof is approximately from 3 to 10 per 100,000 person-years (see Non Patent Literature 4). MALA has a low incidence, but has a mortality rate as high as 50% (see Non Patent Literature 5). An incidence of lactic acidosis in the case where another biguanide, namely phenformin or buformin is taken is much higher than that in the case of metformin, and has led to withdrawal of phenformin and buformin from the market in most countries (see Non Patent Literature 6).

Those facts suggest that metformin will be used for the purposes of treatment of diabetes and cancer, cancer prevention, and anti-aging, and further, will lead to an increase in the number of MALA patients.

Against lactic acidosis, there has been performed symptomatic treatment aimed at reducing production amounts of lactic acid in tissues throughout the entire body through use of supplemental oxygen therapy, a peripheral circulation improving drug, or blood transfusion. However, there has heretofore been no treatment method involving directly reducing a blood lactate level, except for plasma exchange therapy, which can be performed only at a limited number of treatment facilities.

Meanwhile, it is known that a 5-hydroxypyrimidine-4-carboxamide derivative has excellent erythropoietin (EPO) production-enhancing activity, and is effective for treating a disease resulting from a reduction in EPO (Patent Literatures 1 and 2). However, it is not known that the 5-hydroxypyrimidine-4-carboxamide derivative has an effect of preventing or treating lactic acidosis.

### Citation List

### Patent Literature

[PTL 1] WO 2011/049126 A1
[PTL 2] WO 2013/147214 A1

### Non Patent Literature

[NPL 1] Nature 510, 542-546 (2014)
[NPL 2] BMC Biol 12, 82 (2014)
[NPL 3] N Engl J Med 356, 213-215 (2007)
[NPL 4] JAMA 312, 2668-2675 (2014)
[NPL 5] Crit Care 12, R149 (2008)
[NPL 6] Metabolism 65, 20-29 (2016)

### Summary of Invention

### Technical Problem

An object of the present invention is to provide a pharmaceutical composition containing a compound having an excellent effect of preventing or treating lactic acidosis.

### Solution to Problem

The inventors of the present invention have made extensive investigations in order to achieve the above-mentioned object, and as a result, have found that a 5-hydroxypyrimidine-4-carboxamide compound represented by the following general formula (I) or a pharmacologically acceptable salt thereof (hereinafter referred to as compound of the present invention) has an excellent effect of preventing or treating lactic acidosis. Thus, the inventors have completed the present invention.

That is, the present invention is as described below.
[1] A pharmaceutical composition for preventing or treating lactic acidosis, including, as an active ingredient, a compound represented by the general formula (I) or a pharmacologically acceptable salt thereof: 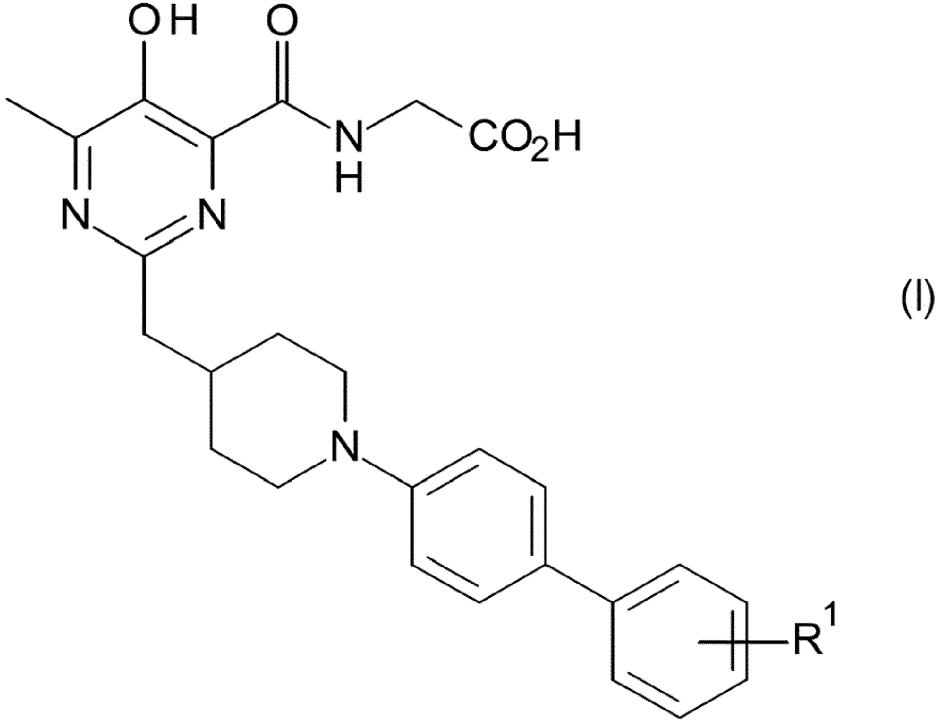 in the general formula (I), R¹ represents a hydroxy C₁ to C₆ alkyl group, a C₂ to C₇ alkanoyl group, a C₂ to C₇ alkanoyl C₁ to C₆ alkyl group, a (C₁ to C₆ alkoxy) carbonyl group, a (C₁ to C₆ alkoxy) carbonyl C₁ to C₆ alkyl group, a carboxy group, or a carboxy C₁ to C₆ alkyl group.
[2] A pharmaceutical composition according to the above-mentioned item 1, wherein R¹ represents a carboxy group, a hydroxymethyl group, a 1-hydroxyethyl group, a 2-hydroxyethyl group, a 2-hydroxypropyl group, a 3-hydroxypropyl group, a 2-hydroxybutyl group, an acetyl group, a methoxycarbonyl group, an ethoxycarbonyl group, a 2-oxopropyl group, a 2-oxobutyl group, a 3-oxobutyl group, a 2-oxopentylgroup, amethoxycarbonylmethylgroup, or a carboxymethyl group.
[3] A pharmaceutical composition according to the above-mentioned item 1, wherein R¹ represents a carboxy group, a hydroxymethyl group, a 1-hydroxyethyl group, a 2-hydroxyethyl group, a 2-hydroxypropyl group, a 3-hydroxypropyl group, a 2-hydroxybutyl group, a 2-oxopropyl group, a 2-oxobutyl group, a 3-oxobutyl group, or a 2-oxopentyl group.
[4] A pharmaceutical composition according to the above-mentioned item 1, wherein the compound having the general formula (I) includes one compound selected from the group consisting of:
   ({[5-hydroxy-2-({1-[4'-(hydroxymethyl)biphenyl-4-yl]piperidin-4-yl}methyl)-6-methylpyrimidin-4-yl]carbonyl}amino)acetic acid;
   ({[5-hydroxy-2-({1-[4'-(2-hydroxypropyl)biphenyl-4-yl]piperidin-4-yl}methyl)-6-methylpyrimidin-4-yl]carbonyl}amino)acetic acid;
   [({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid;
   [({5-hydroxy-2-[(1-{4'-[(2R)-2-hydroxypropyl]biphenyl-4-yl}piperidin-4-yl)methyl]-6-methylpyrimidin-4-yl}carbonyl)amino ]acetic acid;
   ({[5-hydroxy-6-methyl-2-({1-[4'-(2-oxopropyl)biphenyl-4-yl]piperidin-4-yl}methyl)pyrimidin-4-yl]carbonyl}amino)acetic acid; and
   4'-[4-({4-[(carboxymethyl)carbamoyl]-5-hydroxy-6-methylpyrimidin-2-yl}methyl)piperidin-1-yl]biphenyl-4-carboxylic acid.
[5] A pharmaceutical composition according to the above-mentioned item 1, wherein the compound having the general formula (I) includes ({[5-hydroxy-2-({1-[4'-(hydroxymethyl)biphenyl-4-yl]piperidin-4-yl}methyl)-6-methylpyrimidin-4-yl]carbonyl}amino)acetic acid.
[6] A pharmaceutical composition according to the above-mentioned item 1, wherein the compound having the general formula (I) includes [({5-hydroxy-2-[(1-{4'-[(2S)-2-hydroxypropyl]biphenyl-4-yl}pip eridin-4-yl)methyl]-6-methylpyrimidin-4-yl}carbonyl)amino]acet ic acid.
[7] A pharmaceutical composition according to any one of the above-mentioned items 1 to 6, wherein the lactic acidosis includes drug-associated lactic acidosis, renal dysfunction-associated lactic acidosis, shock-associated lactic acidosis, infection-associated lactic acidosis, post-cardiopulmonary resuscitation-associated lactic acidosis, myocardial infarction-associated lactic acidosis, multiple organ failure-associated lactic acidosis, mitochondrial disease-associated lactic acidosis, cancer-associated lactic acidosis, hepatectomy-associated lactic acidosis, severe respiratory disease-associated lactic acidosis, disseminated intravascular coagulation-associated lactic acidosis, severe diabetes-associated lactic acidosis, or alcohol-associated lactic acidosis.
[8] A pharmaceutical composition according to any one of the above-mentioned items 1 to 6, wherein the lactic acidosis includes biguanide-associated lactic acidosis.
[9] A pharmaceutical composition according to any one of the above-mentioned items 1 to 6, wherein the lactic acidosis includes metformin-associated lactic acidosis.
[10] A pharmaceutical composition according to any one of the above-mentioned items 1 to 9, wherein the pharmaceutical composition includes a pharmaceutical composition for oral administration.
[11] A pharmaceutical composition according to any one of the above-mentioned items 1 to 10, wherein the pharmaceutical composition includes a pharmaceutical composition for single administration.
[12] A method of preventing or treating lactic acidosis, including administering the pharmaceutical composition of any one of the above-mentioned items 1 to 11.
[13] A method according to the above-mentioned item 12, wherein the lactic acidosis includes drug-associated lactic acidosis, renal dysfunction-associated lactic acidosis, shock-associated lactic acidosis, infection-associated lactic acidosis, post-cardiopulmonary resuscitation-associated lactic acidosis, myocardial infarction-associated lactic acidosis, multiple organ failure-associated lactic acidosis, mitochondrial disease-associated lactic acidosis, cancer-associated lactic acidosis, hepatectomy-associated lactic acidosis, severe respiratory disease-associated lactic acidosis, disseminated intravascular coagulation-associated lactic acidosis, severe diabetes-associated lactic acidosis, or alcohol-associated lactic acidosis.
[14] A method according to the above-mentioned item 13, wherein the lactic acidosis includes biguanide-associated lactic acidosis.
[15] A method according to the above-mentioned item 13, wherein the lactic acidosis includes metformin-associated lactic acidosis.
[16] A method of preventing or treating lactic acidosis, including administering a compound represented by the general formula (I) or a pharmacologically acceptable salt thereof: 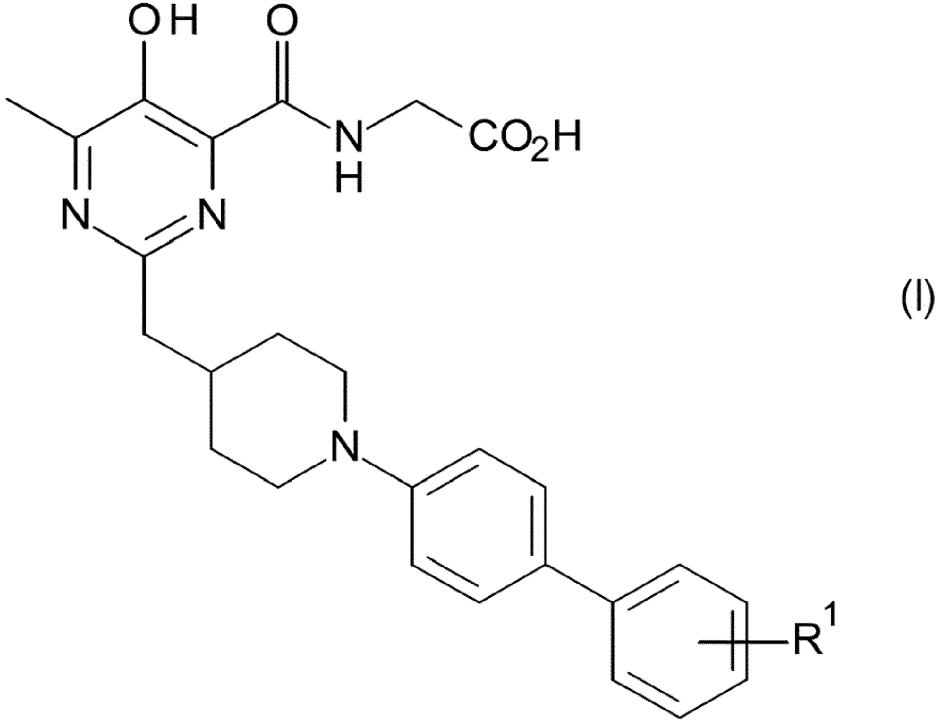 in the general formula (I), R¹ represents a hydroxy C₁ to C₆ alkyl group, a C₂ to C₇ alkanoyl group, a C₂ to C₇ alkanoyl C₁ to C₆ alkyl group, a (C₁ to C₆ alkoxy) carbonyl group, a (C₁ to C₆ alkoxy) carbonyl C₁ to C₆ alkyl group, a carboxy group, or a carboxy C₁ to C₆ alkyl group.
[17] A method according to the above-mentioned item 16, wherein R¹ represents a carboxy group, a hydroxymethyl group, a 1-hydroxyethyl group, a 2-hydroxyethyl group, a 2-hydroxypropyl group, a 3-hydroxypropyl group, a 2-hydroxybutyl group, an acetyl group, a methoxycarbonyl group, an ethoxycarbonyl group, a 2-oxopropyl group, a 2-oxobutyl group, a 3-oxobutyl group, a 2-oxopentyl group, a methoxycarbonylmethyl group, or a carboxymethyl group.
[18] A method according to the above-mentioned item 16, wherein R¹ represents a carboxy group, ahydroxymethyl group, a 1-hydroxyethyl group, a 2-hydroxyethyl group, a 2-hydroxypropyl group, a 3-hydroxypropyl group, a 2-hydroxybutyl group, a 2-oxopropyl group, a 2-oxobutyl group, a 3-oxobutyl group, or a 2-oxopentyl group.
[19] A method according to the above-mentioned item 16, wherein the compound having the general formula (I) includes one compound selected from the group consisting of:
   ({[5-hydroxy-2-({1-[4'-(hydroxymethyl)biphenyl-4-yl]piperidin-4-yl}methyl)-6-methylpyrimidin-4-yl]carbonyl}amino)acetic acid;
   ({[5-hydroxy-2-({1-[4'-(2-hydroxypropyl)biphenyl-4-yl]piperidin-4-yl}methyl)-6-methylpyrimidin-4-yl]carbonyl}amino)acetic acid;
   [({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid;
   [({5-hydroxy-2-[(1-{4'-[(2R)-2-hydroxypropyl]biphenyl-4-yl}piperidin-4-yl)methyl]-6-methylpyrimidin-4-yl}carbonyl)amino]acetic acid;
   ({[5-hydroxy-6-methyl-2-({1-[4'-(2-oxopropyl)biphenyl-4-yl]piperidin-4-yl}methyl)pyrimidin-4-yl]carbonyl}amino)acetic acid; and
   4'-[4-({4-[(carboxymethyl)carbamoyl]-5-hydroxy-6-methylpyrimidin-2-yl}methyl)piperidin-1-yl]biphenyl-4-carboxylic acid.
[20] A method according to any one of the above-mentioned items 16 to 19, wherein the lactic acidosis includes drug-associated lactic acidosis, renal dysfunction-associated lactic acidosis, shock-associated lactic acidosis, infection-associated lactic acidosis, post-cardiopulmonary resuscitation-associated lactic acidosis, myocardial infarction-associated lactic acidosis, multiple organ failure-associated lactic acidosis, mitochondrial disease-associated lactic acidosis, cancer-associated lactic acidosis, hepatectomy-associated lactic acidosis, severe respiratory disease-associated lactic acidosis, disseminated intravascular coagulation-associated lactic acidosis, severe diabetes-associated lactic acidosis, or alcohol-associated lactic acidosis.
[21] A method according to any one of the above-mentioned items 16 to 19, wherein the lactic acidosis includes biguanide-associated lactic acidosis.
[22] A method according to any one of the above-mentioned items 16 to 19, wherein the lactic acidosis includes metformin-associated lactic acidosis.
[23] A method according to any one of the above-mentioned items 16 to 22, wherein the administering includes oral administration.
[24] A method according to any one of the above-mentioned items 16 to 23, wherein the administering includes single administration.
[25] A use of a compound represented by the general formula (I) or a pharmacologically acceptable salt thereof for production of a pharmaceutical composition for preventing or treating lactic acidosis: 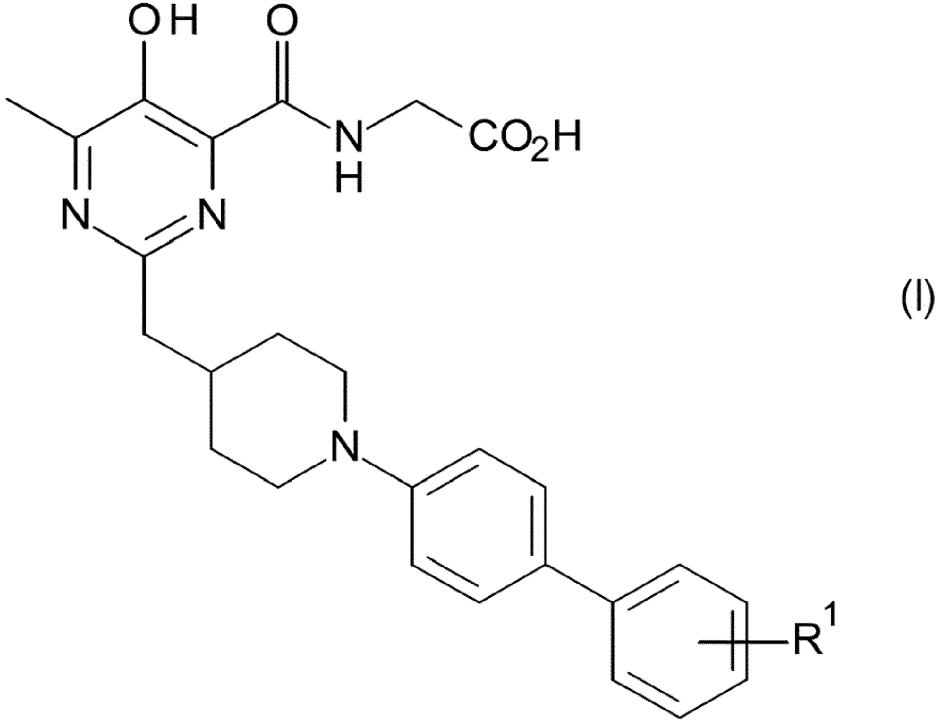 in the general formula (I), R¹ represents a hydroxy C₁ to C₆ alkyl group, a C₂ to C₇ alkanoyl group, a C₂ to C₇ alkanoyl C₁ to C₆ alkyl group, a (C₁ to C₆ alkoxy) carbonyl group, a (C₁ to C₆ alkoxy) carbonyl C₁ to C₆ alkyl group, a carboxy group, or a carboxy C₁ to C₆ alkyl group.
[26] A compound represented by the general formula (I) or a pharmacologically acceptable salt thereof for preventing or treating lactic acidosis: 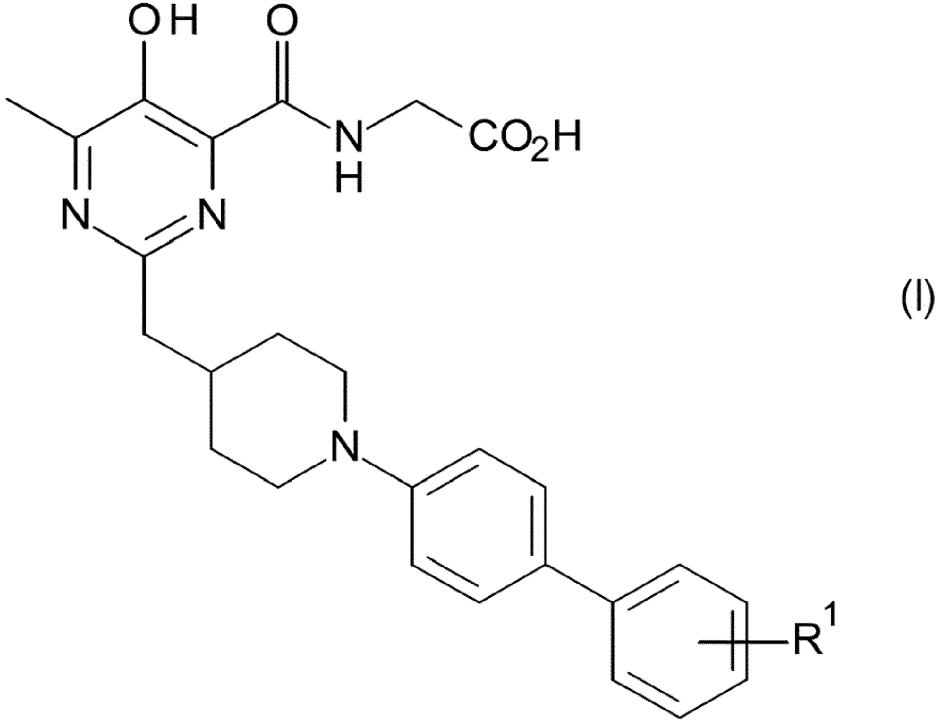 in the general formula (I), R¹ represents a hydroxy C₁ to C₆ alkyl group, a C₂ to C₇ alkanoyl group, a C₂ to C₇ alkanoyl C₁ to C₆ alkyl group, a (C₁ to C₆ alkoxy) carbonyl group, a (C₁ to C₆ alkoxy) carbonyl C₁ to C₆ alkyl group, a carboxy group, or a carboxy C₁ to C₆ alkyl group.

### Advantageous Effects of Invention

The compound of the present invention represented by the general formula (I) has an effect of preventing or treating lactic acidosis, and hence is useful as an active ingredient of a pharmaceutical composition. Accordingly, the pharmaceutical composition for preventing or treating lactic acidosis of the present invention can be used particularly for preventing or treating metformin-associated lactic acidosis and renal dysfunction-associated lactic acidosis.

The pharmaceutical composition for preventing or treating lactic acidosis of the present invention can achieve a treatment result on lactic acidosis through single oral administration, and hence provides great benefits to a medical setting in terms of both treatment effect and medical cost.

### Brief Description of Drawings

FIG. 1 is a graph showing survival rate analysis results of a MALA mouse model administered with ({[5-hydroxy-2-({1-[4'-(hydroxymethyl)biphenyl-4-yl]piperidin-4-yl}methyl)-6-methylpyrimidin-4-yl]carbonyl}amino)acetic acid (hereinafter sometimes referred to as Compound A).
FIG. 2 is a graph showing survival rate analysis results of MALA in a CKD mouse model administered with Compound A.
FIG. 3 is a graph showing survival rate analysis results of MALA in a CKD mouse model administered with [({5-hydroxy-2-[(1-{4'-[(2S)-2-hydroxypropyl]biphenyl-4-yl}pip eridin-4-yl)methyl]-6-methylpyrimidin-4-yl}carbonyl)amino]acet ic acid (hereinafter sometimes referred to as Compound B).

### Description of Embodiments

A pharmaceutical composition for preventing or treating lactic acidosis of the present invention contains, as an active ingredient, a compound represented by the following general formula (I) or a pharmacologically acceptable salt thereof.

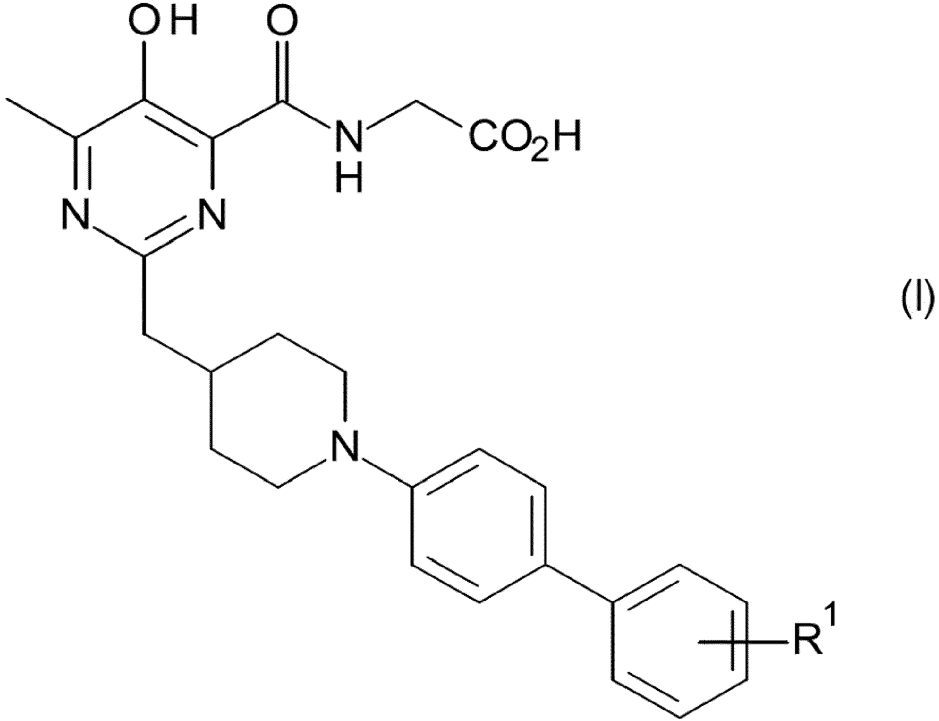

In the general formula (I), R¹ represents a hydroxy C₁ to C₆ alkyl group, a C₂ to C₇ alkanoyl group, a C₂ to C₇ alkanoyl C₁ to C₆ alkyl group, a (C₁ to C₆ alkoxy)carbonyl group, a (C₁ to C₆ alkoxy) carbonyl C₁ to C₆ alkyl group, a carboxy group, or a carboxy C₁ to C₆ alkyl group.

Now, substituents included in the compound represented by the general formula (I) contained in the pharmaceutical composition of the present invention are described.

The "C₁ to C₆ alkyl group" refers to a linear or branched alkyl group having 1 to 6 carbon atoms . Examples thereof may include a methyl group, an ethyl group, a propyl group, an isopropyl group, a butyl group, a sec-butyl group, a tert-butyl group, a pentyl group, an isopentyl group, a 2-methylbutyl group, a neopentyl group, a 1-ethylpropyl group, a hexyl group, a 4-methylpentyl group, a 3-methylpentylgroup, a 2-methylpentylgroup, a 1-methylpentylgroup, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, and a 2-ethylbutyl group.

The "hydroxy C₁ to C₆ alkyl group" in the definition of R¹ refers to a group in which one or more hydrogen atoms (suitably one or two hydrogen atoms) of the "C₁ to C₆ alkyl group" are substituted with a hydroxy group. Examples thereof may include a hydroxymethyl group, a 1-hydroxyethyl group, a 2-hydroxyethyl group, a 1-hydroxypropyl group, a 2-hydroxypropyl group, a 3-hydroxypropyl group, a 2-hydroxy-1,1-dimethylethyl group, a2-hydroxybutylgroup, and a 2-hydroxypentyl group. The hydroxy C₁ to C₆ alkyl group is suitably a hydroxymethyl group, a 1-hydroxyethyl group, a 2-hydroxyethyl group, a 2-hydroxypropyl group, a 3-hydroxypropyl group, or a 2-hydroxybutyl group, more suitably a hydroxymethyl group or a 2-hydroxypropyl group.

The "C₂ to C₇ alkanoyl group" in the definition of R¹ refers to a group in which the "C₁ to C₆ alkyl group" is bonded to a carbonyl group. Examples thereof may include an acetyl group, a propionyl group, a butyryl group, an isobutyryl group, a pentanoyl group, a pivaloyl group, a valeryl group, an isovaleryl group, a hexanoyl group, and a heptanoyl group. The C₂ to C₇ alkanoyl group is suitably an acetyl group.

The "C₂ to C₇ alkanoyl C₁ to C₆ alkyl group" in the definition of R¹ refers to a group in which one hydrogen atom of the "C₁ to C₆ alkyl group" is substituted with the "C₂ to C₇ alkanoyl group." Examples thereof may include a 2-oxopropyl group, a 2-oxobutyl group, a 3-oxobutyl group, a 2-oxopentyl group, a 3-oxopentyl group, and a 4-oxopentyl group. The C₂ to C₇ alkanoyl C₁ to C₆ alkyl group is suitably a 2-oxopropyl group, a 2-oxobutyl group, a 3-oxobutyl group, or a 2-oxopentyl group, more suitably a 2-oxopropyl group.

The "C₁ to C₆ alkoxy group" in the definition of R¹ refers to a group in which the "C₁ to C₆ alkyl group" is bonded to an oxygen atom. Examples thereof may include a methoxy group, an ethoxy group, a n-propoxy group, a n-butoxy group, a s-butoxy group, a tert-butoxy group, and a n-pentoxy group.

The "(C₁ to C₆ alkoxy) carbonyl group" in the definition of R¹ refers to a group in which the "C₁ to C₆ alkoxy group" is bonded to a carbonyl group. Examples thereof may include a methoxycarbonyl group, an ethoxycarbonyl group, a n-propoxycarbonyl group, a n-butoxycarbonyl group, a s-butoxycarbonyl group, a tert-butoxycarbonyl group, and a n-pentoxycarbonyl group. The (C₁ to C₆ alkoxy) carbonyl group is suitably a methoxycarbonyl group or an ethoxycarbonyl group.

The "(C₁ to C₆ alkoxy) carbonyl C₁ to C₆ alkyl group" in the definition of R¹ refers to a group in which the "(C₁ to C₆ alkoxy)carbonyl group" is bonded to the "C₁ to C₆ alkyl group". Examples thereof may include a methoxycarbonylmethyl group, a methoxycarbonylethyl group, an ethoxycarbonylmethyl group, an ethoxycarbonylethyl group, a n-propoxycarbonylmethyl group, a n-propoxycarbonylethyl group, a n-butoxycarbonylmethyl group, and a n-butoxycarbonylethyl group. The (C₁ to C₆ alkoxy) carbonyl C₁ to C₆ alkyl group is suitably a methoxycarbonylmethyl group.

The "carboxy C₁ to C₆ alkyl group" in the definition of R¹ refers to a group in which a carboxy group is bonded to the "C₁ to C₆ alkyl group" . Examples thereof may include a carboxymethyl group, a 1-carboxyethyl group, a 2-carboxyethyl group, a 1-carboxypropyl group, a 2-carboxypropyl group, a 3-carboxypropyl group, a 2-carboxy-1,1-dimethylethyl group, a2-carboxybutylgroup, and a 2-carboxypentyl group. The carboxy C₁ to C₆ alkyl group is suitably a carboxymethyl group.

In the present invention, R¹ preferably represents a carboxy group, a hydroxymethyl group, a 1-hydroxyethyl group, a 2-hydroxyethyl group, a 2-hydroxypropyl group, a 3-hydroxypropyl group, a 2-hydroxybutyl group, an acetyl group, a methoxycarbonyl group, an ethoxycarbonyl group, a 2-oxopropyl group, a 2-oxobutyl group, a 3-oxobutyl group, a 2-oxopentyl group, a methoxycarbonylmethyl group, or a carboxymethyl group, more preferably represents a carboxy group, a hydroxymethyl group, a 1-hydroxyethyl group, a 2-hydroxyethyl group, a 2-hydroxypropyl group, a 3-hydroxypropyl group, a 2-hydroxybutyl group, a methoxycarbonylgroup, anethoxycarbonylgroup, a2-oxopropylgroup, a 2-oxobutyl group, a 3-oxobutyl group, or a 2-oxopentyl group, and still more preferably represents a carboxy group, a hydroxymethyl group, a 2-hydroxypropyl group, or a 2-oxopropyl group.

The compound represented by the general formula (I) of the present invention is preferably one selected from the following compounds or pharmacologically acceptable salts thereof:
({[5-hydroxy-2-({1-[4'-(hydroxymethyl)biphenyl-4-yl]piperidin-4-yl}methyl)-6-methylpyrimidin-4-yl]carbonyl}amino)acetic acid;
({[5-hydroxy-2-({1-[4'-(2-hydroxypropyl)biphenyl-4-yl]piperidin-4-yl}methyl)-6-methylpyrimidin-4-yl]carbonyl}amino)acetic acid;
[({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid;
[({5-hydroxy-2-[(1-{4'-[(2R)-2-hydroxypropyl]biphenyl-4-yl}piperidin-4-yl)methyl]-6-methylpyrimidin-4-yl}carbonyl)amino]acetic acid;
({ [5-hydroxy-6-methyl-2-({1-[4'-(2-oxopropyl)biphenyl-4-yl]piperidin-4-yl}methyl)pyrimidin-4-yl]carbonyl}amino)acetic acid; and
4'-[4-({4-[(carboxymethyl)carbamoyl]-5-hydroxy-6-methylpyrimidin-2-yl}methyl)piperidin-1-yl]biphenyl-4-carboxylic acid.

When the compound represented by the general formula (I) of the present invention has an asymmetric carbon atom, optical isomers may exist. The present invention encompasses those isomers separated from each other (e.g., an enantiomer or a diastereomer), or a mixture (e.g., a racemic mixture or a diastereomer mixture).

When the compound represented by the general formula (I) of the present invention has a basic group, such as an amino group, a pharmacologically acceptable acid addition salt thereof may be formed as desired. Examples of such acid addition salt may include: hydrohalides, such as a hydrofluoride, a hydrochloride, a hydrobromide, and a hydroiodide; inorganic acid salts, such as a nitrate, a perchlorate, a sulfate, and a phosphate; lower alkanesulfonates, such as a methanesulfonate, a trifluoromethanesulfonate, and an ethanesulfonate; aryl sulfonates, such as a benzenesulfonate and a p-toluenesulfonate; organic acid salts, such as an acetate, a malate, a fumarate, a succinate, a citrate, a tartrate, an oxalate, and a maleate; and amino acid salts, such as an ornithinate, a glutamate, and an aspartate. Of those, hydrohalides and organic acid salts are preferred.

When the compound represented by the general formula (I) of the present invention has an acidic group, such as a carboxy group, it is generally possible to form a pharmacologically acceptable base addition salt thereof. Examples of such base addition salt may include: alkali metal salts, such as a sodium salt, a potassium salt, and a lithium salt; alkaline earth metal salts, such as a calcium salt and a magnesium salt; inorganic salts, such as an ammonium salt; and organic amine salts, such as a dibenzylamine salt, a morpholine salt, a phenylglycine alkyl ester salt, an ethylenediamine salt, an N-methylglucamine salt, a diethylamine salt, a triethylamine salt, a cyclohexylamine salt, a dicyclohexylamine salt, an N,N'-dibenzylethylenediamine salt, a diethanolamine salt, an N-benzyl-N-(2-phenylethoxy)amine salt, a piperazine salt, a tetramethylammonium salt, and a tris(hydroxymethyl)aminomethane salt.

The compound represented by the general formula (I) of the present invention may exist in an unsolvated form or as a solvate . The solvate is not particularly limited as long as the solvate is pharmacologically acceptable, but specifically, a hydrate, an ethanolate, or the like is preferred.

The compound represented by the general formula (I) of the present invention may be produced by a method described in, for example, WO2011/049126A1 (Patent Literature 1) or WO2013/147214 A1 (Patent Literature 2).

The "lactic acidosis" in the preventing or treating lactic acidosis according to the present invention refers to a pathological condition in which a rise in serum lactate level, a reduction in arterial blood pH, a rise in blood PaCO₂ (arterial carbon dioxide partial pressure), a reduction in bicarbonate ions (HCO₃⁻), an increase in anion gap, an increase in lactate/pyruvate ratio, and the like are found. Diagnosis of lactic acidosis is generally performed by measuring a blood lactate level and a pH through blood gas analysis. A case in which arterial blood gas analysis finds a pH of less than 7.35 and a blood lactate concentration of 5 mmol/L (45 mg/dL) or more is diagnosed with lactic acidosis.

The "lactic acidosis" in the preventing or treating lactic acidosis according to the present invention is not particularly limited, but examples thereof may include drug-associated lactic acidosis {e.g., biguanide-associated lactic acidosis (more specifically, metformin-associated lactic acidosis or buformin-associated lactic acidosis)}, renal dysfunction-associated lactic acidosis (e.g., chronic renal failure-associated lactic acidosis), shock-associated lactic acidosis (e.g., lactic acidosis associated with circulatory failure, bleeding, or the like, more specifically, lactic acidosis associated with circulatory failure, bleeding, or the like due to insufficient cardiac output in a patient with dilated cardiomyopathy), infection (sepsis)-associated lactic acidosis (e.g., pneumonia/peritonitis-associated lactic acidosis), post-cardiopulmonary resuscitation-associated lactic acidosis, myocardial infarction-associated lactic acidosis, multiple organ failure-associated lactic acidosis, mitochondrial disease-associated lactic acidosis (e.g., mitochondrial encephalomyopathy-associated lactic acidosis), cancer-associated lactic acidosis, hepatectomy-associated lactic acidosis, severe respiratory disease-associated lactic acidosis, disseminated intravascular coagulation-associated lactic acidosis, severe diabetes-associated lactic acidosis, and alcohol-associated lactic acidosis. Of those, metformin-associated lactic acidosis and renal dysfunction-associated lactic acidosis are particularly preferred.

The pharmaceutical composition for preventing or treating lactic acidosis of the present invention is used for a mammal (e.g., a human, a horse, cattle, or a pig) or a bird (e.g., a chicken), and is prepared using an additive, such as a carrier or an excipient, which is used for a general pharmaceutical composition (formulation). The administration of the pharmaceutical composition of the present invention may be oral administration in the form of a tablet, a pill, a capsule, a granule, a powder, a solution, or the like, or parenteral administration in the form of an injection (e.g., an intravenous injection or an intramuscular injection), a suppository, a transdermal preparation, a transnasal preparation, an inhalant, or the like.

A solid formulation for the oral administration according to the present invention may be a tablet, a powder, a granule, or the like. Such formulation is produced by mixing one or more active substances with, for example, an inert excipient, lubricant, disintegrant, or dissolution aid in accordance with a conventional method. The excipient may be, for example, lactose, cellulose, mannitol, or glucose. The lubricant may be, for example, magnesium stearate. The disintegrant may be, for example, sodium carboxymethyl starch. The tablet or the pill may be sugar-coated or coated with a gastrosoluble or enteric coating agent as required.

A liquid formulation for the oral administration may be, for example, a pharmaceutically acceptable emulsion, solution, suspension, syrup, or elixir. Such formulation contains a generally used inert solvent (e.g., purified water or ethanol), and may further contain a solubilizing agent, a wetting agent, a suspending agent, a sweetening agent, a taste-masking agent, an aromatic, or an antiseptic.

An injection for the parenteral administration may be a sterile, aqueous or non-aqueous solution, suspension, or emulsion. An aqueous solvent for the injection may be, for example, distilled water or physiological saline. A non-aqueous solvent for the injection may be, for example, propylene glycol, polyethylene glycol, a plant oil, such as an olive oil, an alcohol, such as ethanol, or Polysorbate 80 (Japanese Pharmacopoeia name). Such formulation may further contain a tonicity agent, an antiseptic, a wetting agent, an emulsifier, a dispersant, a stabilizer, or a dissolution aid. Those formulations may be sterilized by, for example, filtration with a bacteria-retaining filter, blending of a germicide, or radiation irradiation. In addition, a composition obtained by dissolving or suspending a sterile solid composition in sterile water or a solvent for injection before use may also be used as the above-mentioned formulation.

The dose and number of times of administration of the pharmaceutical composition of the present invention are determined as appropriate for individual cases in consideration of, for example, symptoms, and the age or sex of an administration target. A single dose for an adult in the case of oral administration is generally from 0.001 mg/kg body weight to 100 mg/kg body weight, and a single dose for an adult in the case of intravenous administration is generally from 0.0001 mg/kg body weight to 10 mg/kg body weight. The number of times of administration is generally from 1 to 6 per day or from 1 per day to 1 in 7 days. It is also preferred that administration to a patient receiving dialysis be performed once before or after each dialysis that the patient receives (suitably before the dialysis). In particular, the pharmaceutical composition of the present invention is considered to have an effect of reducing a blood lactate level, and moreover, is considered to have an effect through oral administration, and even single administration on the basis of the results of Examples.

The pharmaceutical composition for preventing or treating lactic acidosis of the present invention may be used in combination with supplemental oxygen therapy, blood transfusion, plasma exchange therapy, or the like.

The pharmaceutical composition for preventing or treating lactic acidosis of the present invention may be administered together with a peripheral circulation improving drug. Examples of the peripheral circulation improving drug include nitroglycerin and prostaglandin formulations.

The present invention also encompasses a compound represented by the general formula (I) or a pharmacologically acceptable salt thereof for preventing or treating lactic acidosis: 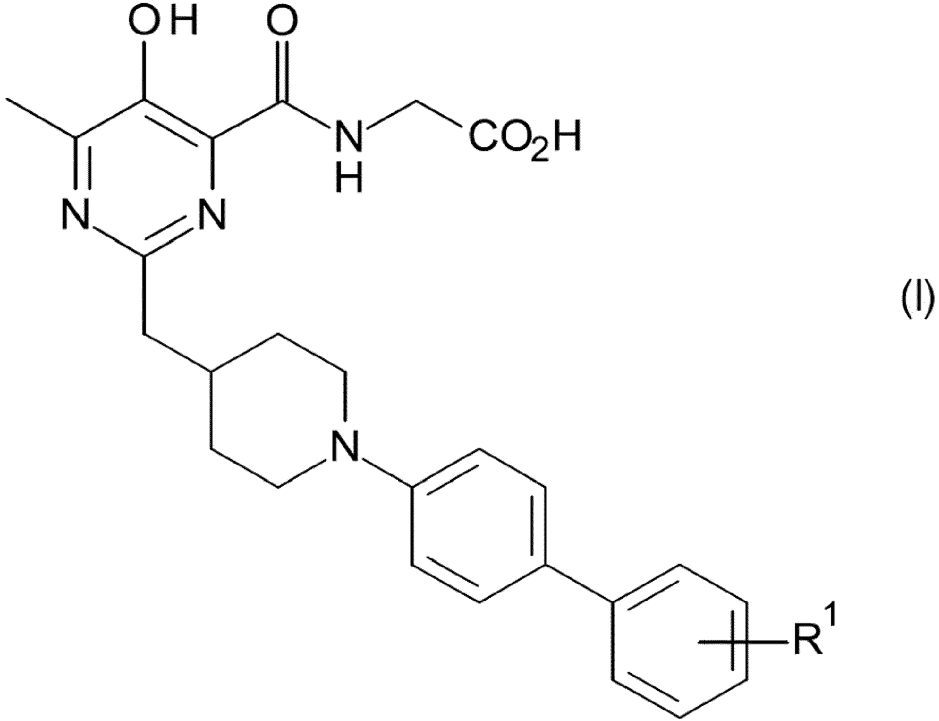 in the general formula (I), R¹ represents a hydroxy C₁ to C₆ alkyl group, a C₂ to C₇ alkanoyl group, a C₂ to C₇ alkanoyl C₁ to C₆ alkyl group, a (C₁ to C₆ alkoxy) carbonyl group, a (C₁ to C₆ alkoxy) carbonyl C₁ to C₆ alkyl group, a carboxy group, or a carboxy C₁ to C₆ alkyl group.

The present invention also encompasses a use of a compound represented by the general formula (I) or a pharmacologically acceptable salt thereof for production of a pharmaceutical composition for preventing or treating lactic acidosis: 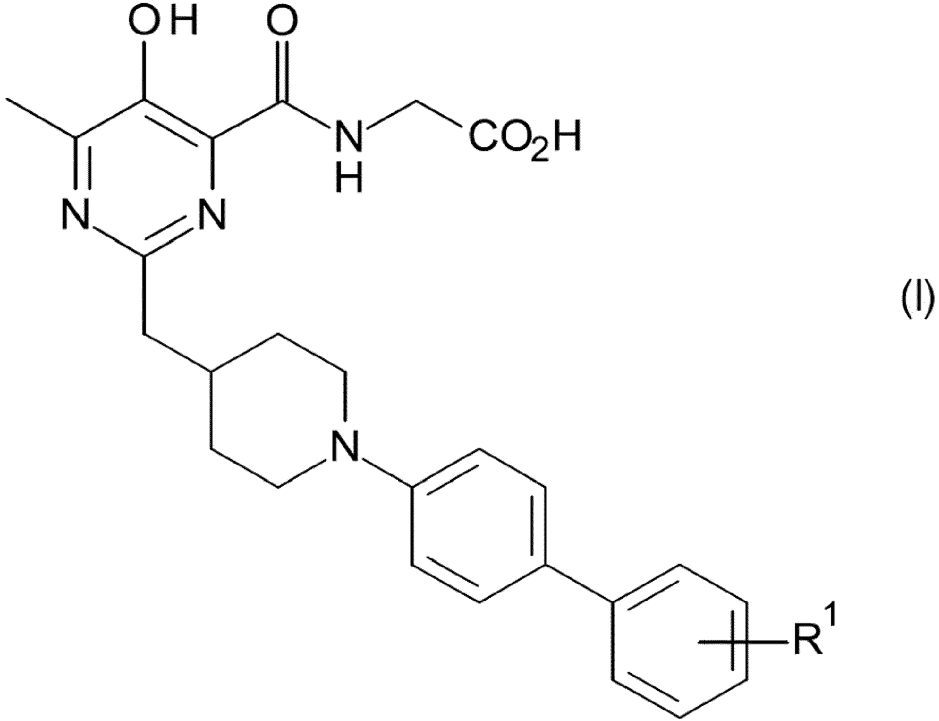 in the general formula (I), R¹ represents a hydroxy C₁ to C₆ alkyl group, a C₂ to C₇ alkanoyl group, a C₂ to C₇ alkanoyl C₁ to C₆ alkyl group, a (C₁ to C₆ alkoxy) carbonyl group, a (C₁ to C₆ alkoxy) carbonyl C₁ to C₆ alkyl group, a carboxy group, or a carboxy C₁ to C₆ alkyl group.

Now, the present invention is further described in detail by describing Examples and Test Examples, but the scope of the present invention is not limited thereto . All experiments have been approved by the Animal Care and Utilization Committee of the Keio University School of Medicine (#11050-1).

### Examples

### (Materials and Methods)

All mice used in Examples were 7- to 13-week-old wild-type C57BL/6 male mice obtained from CLEA Japan, Inc. The mice were fed ad libitum on a 12-hour light/12-hour dark cycle in a specific pathogen-free facility.

In statistical analysis in Examples, power calculation for determining a sample size was not performed. Mice that died of lactic acidosis before treatment with a vehicle or Compound A were excluded from mice to be analyzed. For a comparison of normally distributed data between groups, analysis was performed using unpaired Student's t-test. A difference in survival rate was analyzed by log-rank (Mantel-Cox) test through use of GraphPad Prism software.

### (Example 1)

### Production of ({[5-hydroxy-2-({1-[4'-(hydroxymethyl)biphenyl-4-yl]piperidin-4-yl}methyl)-6-methylpyrimidin-4-yl]carbonyl}amino)acetic acid (Compound A)

Compound A was produced in accordance with the method of Example 1 of WO 2011/049126 A1 (Patent Literature 1). The produced compound was identified as Compound A by ¹H-NMR and mass spectrometry.

### (Example 2)

### Production of [({5-hydroxy-2-[(1-{4'-[(2S)-2-hydroxypropyl]biphenyl-4-yl}pip eridin-4-yl)methyl]-6-methylpyrimidin-4-yl}carbonyl)amino]acet ic acid (Compound B)

Compound B was produced in accordance with the method of Example 45 of WO 2011/049126 A1 (Patent Literature 1). The produced compound was identified as Compound B by ¹H-NMR and mass spectrometry.

### (Test Example 1)

### Effect of Administration of Compound A on Metformin-associated Lactic Acidosis (MALA) model

Metformin promotes the accumulation of nicotinamide adenine dinucleotide (NADH), which is not desirable for conversion of lactate to pyruvate by lactate dehydrogenase (LDH), to thereby cause MALA. MALA is a serious symptom with a high mortality rate particularly among elderly patients and patients with renal failure. In view of this, in this Test Example, the improving effect of Compound A on the survival rate of a MALA model was verified.

In the morning and evening of day 1, and the morning of day 2, 0.25 mg/g body weight of metformin dissolved in distilled water was orally administered to 8-week-old wild-type C57BL/6 male mice to produce MALA model mice. Immediately after the metformin administration to the model mice on day 2, a vehicle (0.5% methyl cellulose; 64625; Sigma-Aldrich) or 30 mg/kg body weight of Compound A (produced in Example 1) was orally administered. After 4 hours from the oral administration, the model mice were administered with 0.4 mg/g body weight of lactic acid dissolved in 0.9% NaCl by intraperitoneal injection. Kaplan-Meier survival analysis was performed for those mice.

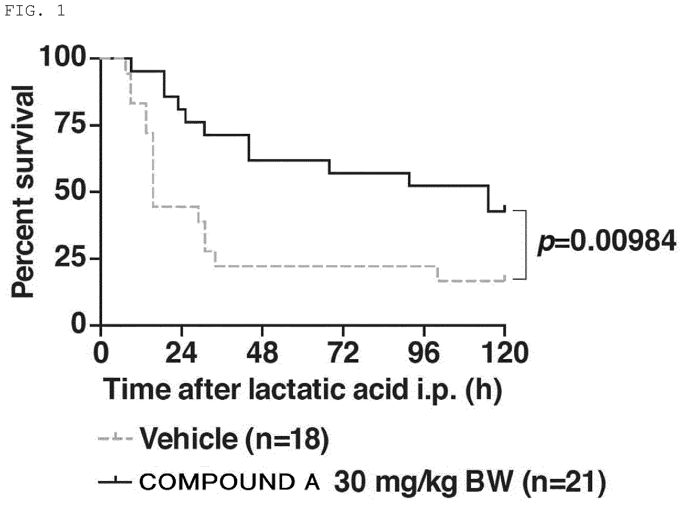

As a result of the survival analysis, the MALA model mice administered with Compound A showed a significantly high survival rate as compared to the MALA model mice administered with the vehicle alone (FIG. 1). More specifically, the MALA model mice administered with Compound A had an about 1.8-fold survival rate after 24 hours, an about 2.8-fold survival rate after 48 hours, an about 2.6-fold survival rate after 72 hours, an about 2.4-fold survival rate after 96 hours, and an about 2.6-fold survival rate after 120 hours, as compared to the MALA model mice administered with the vehicle alone.

The results verified that the compound represented by the general formula (I) of the present invention (in particular, Compound A) had an effect on the prevention or treatment of metformin-associated lactic acidosis.

### (Test Example 2)

### Effect of Administration of Compound A on MALA-complicated Chronic Kidney Disease (CKD) Model Mice

Metformin is found to pose a risk of lactic acidosis in CKD patients or elderly individuals having high serum creatinine values, and hence is contraindicated (reference: Non Patent Literature 4) . In view of this, in this Test Example, the improving effect of Compound A on the survival rate of a MALA-complicated CKD model was verified.

7-Week-old wild-type C57BL/6 male mice were fed ad libitum with a standard or 0.2% adenine feed for 6 weeks. The population fed with the 0.2% adenine feed was used as CKD model mice.

Further, the CKD model mice were administered with 0.5 mg/g body weight of metformin on day 1 to produce a MALA-complicated CKD mouse model. After 4 hours from the metformin administration, a whole blood sample (5 µl) was collected from the tail vein, and a blood lactate level was measured using Lactate Pro Test Meter and Lactate Pro Test Strip (Cycle Classic Imports). Mice having a blood lactate level of more than 8 mM were orally administered with a vehicle (0.5% methyl cellulose; 64625; Sigma-Aldrich) or 10 mg/kg body weight of Compound A. The experiment was repeated the next day for mice whose lactate level did not satisfy the criterion. Kaplan-Meier survival analysis was performed for those mice.

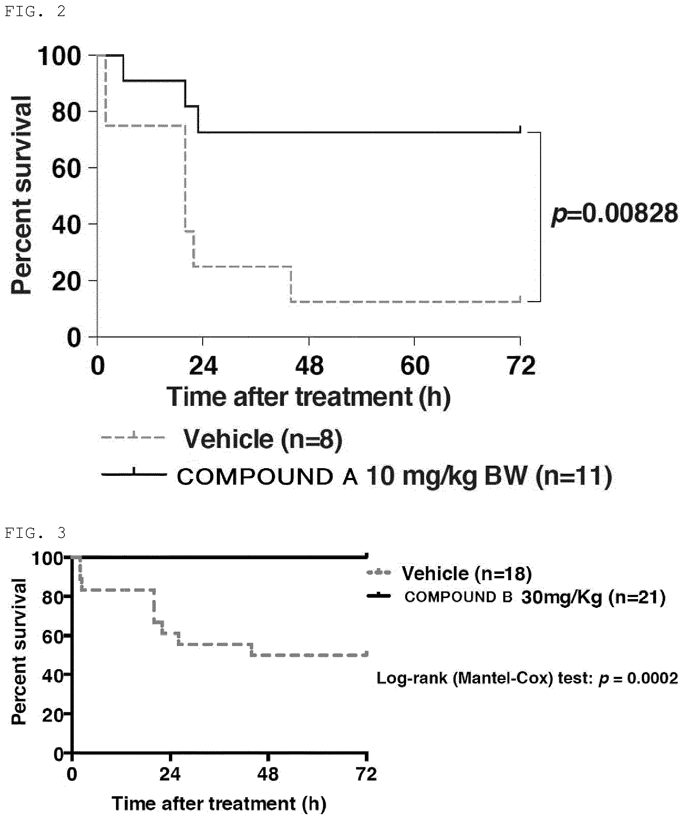

As a result of the survival analysis, the MALA-complicated CKD model mice administered with Compound A showed a significantly high survival rate as compared to the MALA-complicated CKD model mice administered with the vehicle alone (FIG. 2). More specifically, the MALA-complicated CKD model mice administered with Compound A had an about 2.9-fold survival rate after 24 hours, an about 5.8-fold survival rate after 48 hours, and an about 5.8-fold survival rate after 72 hours, as compared to the mice administered with vehicle alone.

The results verified that the compound represented by the general formula (I) of the present invention (in particular, Compound A) had an effect on the prevention agent or treatment of chronic kidney disease-associated lactic acidosis.

### (Test Example 3)

### Effect of Administration of Compound B on MALA-complicated Chronic Kidney Disease (CKD) Model Mice

7-Week-old wild-type C57BL/6 male mice were fed ad libitum with a standard or 0.2% adenine feed for 6 weeks. The population fed with the 0.2% adenine feed was used as CKD model mice.

Further, the CKD model mice were administered with 0.5 mg/g body weight of metformin on day 1 to produce a MALA-complicated CKD mouse model. After 4 hours from the metformin administration, a whole blood sample (5 µl) was collected from the tail vein, and a blood lactate level was measured using Lactate Pro Test Meter and Lactate Pro Test Strip (Cycle Classic Imports). Mice having a blood lactate level of more than 8 mM were orally administered with a vehicle (0.5% methyl cellulose; 64625; Sigma-Aldrich) or 30 mg/kg body weight of Compound B. The experiment was repeated the next day for mice whose lactate level did not satisfy the criterion. Kaplan-Meier survival analysis was performed for those mice. The results are shown in FIG. 3.

As a result of the survival analysis, the MALA-complicated CKD model mice administered with Compound B showed a significantly high survival rate as compared to the MALA-complicated CKD model mice administered with the vehicle alone (FIG. 3). More specifically, the MALA-complicated CKD model mice administered with Compound B had an about 1.7-fold survival rate after 24 hours and about 2.0-fold survival rates after 48 hours and after 72 hours, as compared to the mice administered with vehicle alone.

The results verified that the compound represented by the general formula (I) of the present invention (in particular, Compound B) had an effect on the prevention agent or treatment of chronic kidney disease-associated lactic acidosis.

### (Formulation Example)

### Formulation Example 1 (Tablet)

100 mg of the compound of one of Examples in the form of powder, 124 mg of lactose, 25 mg of cellulose, and 1 mg of magnesium stearate are mixed, and the mixture is tableted with a tableting machine into tablets weighing 200 mg per tablet. The tablets may be sugar-coated as required.

As described above, it has been demonstrated that the compound represented by the general formula (I) of the present invention remarkably improves survival rates for fatal metformin-associated lactic acidosis and chronic kidney disease-associated lactic acidosis through single oral administration.

According to those results, the pharmaceutical composition for preventing or treating lactic acidosis of the present invention can be utilized for novel prevention or treatment of lactic acidosis .

### Industrial Applicability

The pharmaceutical composition for preventing or treating lactic acidosis of the present invention can be used for preventing or treating drug-associated lactic acidosis, renal dysfunction-associated lactic acidosis, shock-associated lactic acidosis, infection-associated lactic acidosis, post-cardiopulmonary resuscitation-associated lactic acidosis, myocardial infarction-associated lactic acidosis, multiple organ failure-associated lactic acidosis, mitochondrial disease-associated lactic acidosis, cancer-associated lactic acidosis, hepatectomy-associated lactic acidosis, severe respiratory disease-associated lactic acidosis, disseminated intravascular coagulation-associated lactic acidosis, severe diabetes-associated lactic acidosis, or alcohol-associated lactic acidosis, in particular, metformin-associated lactic acidosis and renal dysfunction-associated lactic acidosis.

## Claims

1. A pharmaceutical composition for preventing or treating lactic acidosis, comprising, as an active ingredient, a compound represented by the general formula (I) or a pharmacologically acceptable salt thereof: 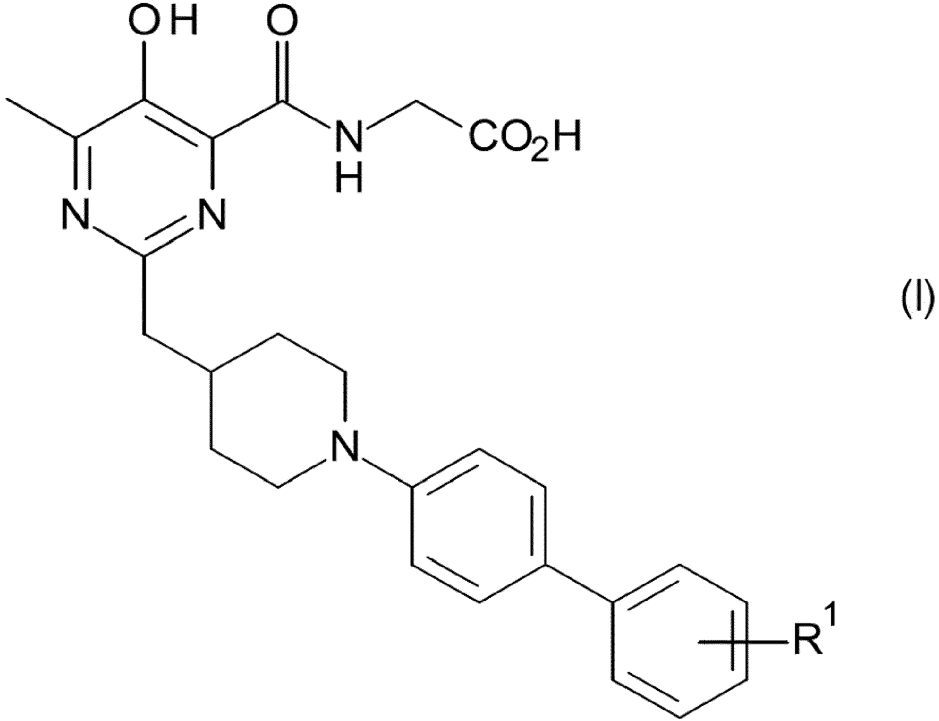 in the general formula (I), R¹ represents a hydroxy C₁ to C₆ alkyl group, a C₂ to C₇ alkanoyl group, a C₂ to C₇ alkanoyl C₁ to C₆ alkyl group, a (C₁ to C₆ alkoxy) carbonyl group, a (C₁ to C₆ alkoxy) carbonyl C₁ to C₆ alkyl group, a carboxy group, or a carboxy C₁ to C₆ alkyl group.

2. A pharmaceutical composition according to claim 1, wherein R¹ represents a carboxy group, a hydroxymethyl group, a 1-hydroxyethyl group, a 2-hydroxyethyl group, a 2-hydroxypropyl group, a 3-hydroxypropyl group, a 2-hydroxybutyl group, an acetyl group, a methoxycarbonyl group, an ethoxycarbonyl group, a 2-oxopropyl group, a 2-oxobutyl group, a 3-oxobutyl group, a 2-oxopentyl group, a methoxycarbonylmethyl group, or a carboxymethyl group.

3. A pharmaceutical composition according to claim 1, wherein R¹ represents a carboxy group, a hydroxymethyl group, a 1-hydroxyethyl group, a 2-hydroxyethyl group, a 2-hydroxypropyl group, a 3-hydroxypropyl group, a 2-hydroxybutyl group, a 2-oxopropyl group, a 2-oxobutyl group, a 3-oxobutyl group, or a 2-oxopentyl group.

4. A pharmaceutical composition according to claim 1, wherein the compound having the general formula (I) comprises one compound selected from the group consisting of:
({[5-hydroxy-2-({1-[4'-(hydroxymethyl)biphenyl-4-yl]piperidin-4-yl}methyl)-6-methylpyrimidin-4-yl]carbonyl}amino)acetic acid;
({[5-hydroxy-2-({1-[4'-(2-hydroxypropyl)biphenyl-4-yl]piperidin-4-yl}methyl)-6-methylpyrimidin-4-yl]carbonyl}amino)acetic acid;
[({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid;
[({5-hydroxy-2-[(1-{4'-[(2R)-2-hydroxypropyl]biphenyl-4-yl}piperidin-4-yl)methyl]-6-methylpyrimidin-4-yl}carbonyl)amino]acetic acid;
({[5-hydroxy-6-methyl-2-({1-[4'-(2-oxopropyl)biphenyl-4-yl]piperidin-4-yl}methyl)pyrimidin-4-yl]carbonyl}amino)acetic acid; and
4'-[4-({4-[(carboxymethyl)carbamoyl]-5-hydroxy-6-methylpyrimidin-2-yl}methyl)piperidin-1-yl]biphenyl-4-carboxylic acid.

5. A pharmaceutical composition according to claim 1, wherein the compound having the general formula (I) comprises ({[5-hydroxy-2-({1-[4'-(hydroxymethyl)biphenyl-4-yl]piperidin-4-yl}methyl)-6-methylpyrimidin-4-yl]carbonyl}amino)acetic acid.

6. A pharmaceutical composition according to claim 1, wherein the compound having the general formula (I) comprises [({5-hydroxy-2-[(1-{4'-[(2S)-2-hydroxypropyl]biphenyl-4-yl}pip eridin-4-yl)methyl]-6-methylpyrimidin-4-yl}carbonyl)amino]acet ic acid.

7. A pharmaceutical composition according to any one of claims 1 to 6, wherein the lactic acidosis comprises drug-associated lactic acidosis, renal dysfunction-associated lactic acidosis, shock-associated lactic acidosis, infection-associated lactic acidosis, post-cardiopulmonary resuscitation-associated lactic acidosis, myocardial infarction-associated lactic acidosis, multiple organ failure-associated lactic acidosis, mitochondrial disease-associated lactic acidosis, cancer-associated lactic acidosis, hepatectomy-associated lactic acidosis, severe respiratory disease-associated lactic acidosis, disseminated intravascular coagulation-associated lactic acidosis, severe diabetes-associated lactic acidosis, or alcohol-associated lactic acidosis.

8. A pharmaceutical composition according to any one of claims 1 to 6, wherein the lactic acidosis comprises biguanide-associated lactic acidosis.

9. A pharmaceutical composition according to any one of claims 1 to 6, wherein the lactic acidosis comprises metformin-associated lactic acidosis.

10. A pharmaceutical composition according to any one of claims 1 to 9, wherein the pharmaceutical composition comprises a pharmaceutical composition for oral administration.

11. A pharmaceutical composition according to any one of claims 1 to 10, wherein the pharmaceutical composition comprises a pharmaceutical composition for single administration.

12. A method of preventing or treating lactic acidosis, comprising administering the pharmaceutical composition of any one of claims 1 to 11.

13. A method according to claim 12, wherein the lactic acidosis comprises drug-associated lactic acidosis, renal dysfunction-associated lactic acidosis, shock-associated lactic acidosis, infection-associated lactic acidosis, post-cardiopulmonary resuscitation-associated lactic acidosis, myocardial infarction-associated lactic acidosis, multiple organ failure-associated lactic acidosis, mitochondrial disease-associated lactic acidosis, cancer-associated lactic acidosis, hepatectomy-associated lactic acidosis, severe respiratory disease-associated lactic acidosis, disseminated intravascular coagulation-associated lactic acidosis, severe diabetes-associated lactic acidosis, or alcohol-associated lactic acidosis.

14. A method according to claim 13, wherein the lactic acidosis comprises biguanide-associated lactic acidosis.

15. A method according to claim 13, wherein the lactic acidosis comprises metformin-associated lactic acidosis.
